(19) Europäisches Patentamt
European Patent Office
Office européen des brevets

(11) Publication number: **0 009 944**
**B1**

(12) # EUROPEAN PATENT SPECIFICATION

(45) Date of publication of patent specification: **18.05.83**

(51) Int. Cl.³: **A 61 K 31/13,** C 07 C 87/22 //C07C103/34

(21) Application number: **79302045.4**

(22) Date of filing: **28.09.79**

(54) **Lysosomotropic fluorinated amine therapeutic agents and compositions containing them.**

(30) Priority: **02.10.78 US 947374**

(43) Date of publication of application:
**16.04.80 Bulletin 80/8**

(45) Publication of the grant of the patent:
**18.05.83 Bulletin 83/20**

(84) Designated Contracting States:
**AT BE CH DE FR GB IT LU NL SE**

(56) References cited:
**EP - A - 0 000 951**
**FR - A - 2 340 729**
**FR - M - 1 528**
**GB - A - 1 364 312**
**US - A - 3 718 651**
**US - A - 3 840 542**

**BIOCHEMICAL PHARMACOLOGY, vol. 23, no. 18, 1974, C. DE DUVE et al. "Lysosomotropic agents", pages 2495—2531**

(73) Proprietor: **MERCK & CO. INC.**
**126, East Lincoln Avenue P.O. Box 2000**
**Rahway New Jersey 07065 (US)**

(72) Inventor: **Firestone, Raymond A.**
**60 Hunter Avenue**
**Fanwood, New Jersey 07023 (US)**

(74) Representative: **Crampton, Keith John Allen et al,**
**D YOUNG & CO 10 Staple Inn**
**London WC1V 7RD (GB)**

(56) References cited:
**JOURNAL OF DAIRY SCIENCE, vol. 62, no. 4, April 1979, M. D. CULLER et al. "Mastitis: I. In Vitro Antimicrobial Activity of Alkyl Amines Against Mastitic Bacteria", pages 584—595**
**JOURNAL OF MEDICINAL CHEMISTRY, vol. 22, no. 9, September 1979 R. A. FIRESTONE and J. M. PISANO. "Lysosomotropic agents. 1. Synthesis and Cytotoxic Action of Lososomotropic Detergents"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 009 944 B1

Courier Press, Leamington Spa, England.

# Lysosomotropic fluorinated amine therapeutic agents and compositions containing them

This invention relates to compositions useful in the control of fertility, in the inhibition of malignant cell growth, and in the control of other diseases which accumulate macrophages at the disease site.

Lysosomotropic substances have been designated as substances which are selectively taken up by lysosomes regardless of their chemical nature or mechanism of uptake. It has been suggested that the property of lysosomotropism is useful in the preparation of therapeutic agents having useful biological properties. These substances can enter the lysosomes preferentially and exert their biological influence because of their own unique biological activity. They also can be coupled with active drugs and, following preferential uptake by lysosomes, can be hydrolysed within the lysosome with release of the active drug at the target area. Thus, in one method of using the lysosomotropic agent DNA, a complex of DNA with the potent cytotoxic agent daunorubicin was formed. It was then found that the complex was preferentially taken up by the tumour cells and that the DNA was preferentially degraded by lysosomes leaving the active daunorubicin with the lysosome. A drawback of this particular method is that the toxic dose is very close to the therapeutic dose which makes the margin of safety very narrow.

The present invention is concerned with the inhibition of lysosome-bearing cells and involves the use of new compositions and new pharmaceutical formulations useful in inhibiting the function of lysosome-bearing cells.

Still more specifically, the present invention provides a pharmaceutical composition comprising a pharmaceutical carrier and a lysosomotropic substance having a pK of from 4.5 to 9, preferably 5 to 8, that is an alkyl amine compound of the formula:

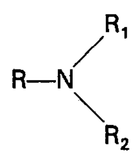

in which R is a $C_{8-30}$ alkyl group, $R^1$ a hydrogen atom or an alkyl group and $R^2$ is a $C_{2-3}$ fluorinated alkyl group in which the fluorine atom(s) is/are attached to its terminal carbon atom. R is preferably $C_{8-18}$ alkyl and $R_2$ is preferably a 2,2-difluoroethyl or 2,2,2-trifluoroethyl group.

The above compounds are novel and constitute another embodiment of the present invention.

The compositions of the present invention are useful in selectively killing or altering the function of lysosome-bearing cells, and they are also useful as systems for the delivery of an active drug to the disease site where the active drug is released by lysosomal enzymatic hydrolysis. Thus, the compositions of this invention are useful as antifertility agents. As antifertility agents, the active principle is incorporated into vaginal or other contraceptive creams, jellies, or foams, e.g. in a concentration of from 0.1% to 10%, preferably 1—10%. The formulation prepared in this manner is administered intravaginally and contact of the sperm with the composition of the present invention incorporates the active principle into the acrosome of the sperm cells, thus rendering them infertile.

The compositions of the present invention are also useful in inhibiting the growth of malignant tumour cells. For this purpose, the compositions of the present invention are administered either orally or by injection in a dosage schedule of 25—500 mg/kg/day.

The compositions of the present invention are also useful in combating infection caused by resistant microorganisms, which are sequestered within lysosomes and thus unavailable to attack by antibiotics. The lysosomotropic compositions of the present invention comprise an antibiotic incorporating a weakly basic amine group and a peptide linked to a carboxylic function of the antibiotic through an amide function. Such compositions are incorporated into the cell lysosome where they are hydrolysed by lysosomal enzymes, thus releasing the active antibiotic principle to attack the infecting microorganism.

Certain of the compositions of the present invention are useful in the treatment of inflammation. Thus cholesterol is rendered antiinflammatory by incorporating within it a lysosomotropic amine such as morpholine or trifluoroethylamine.

The compounds of this invention can be administered either topically or systemically (i.e., intravenously, subcutaneously, intramuscularly, orally, rectally, by aerosolization, or in the form of sterile implants for a long action.

The pharmaceutical compositions can be sterile, injectable suspensions or solutions, or solid, orally administrable, pharmaceutically acceptable tablets or capsules; the compositions can also be intended for sub-lingual administration, or for suppository use. It is especially advantageous to formulate compositions in dosage unit form for ease and economy of administration and uniformity of dosage. "Dosage unit form" as a term used herein refers to physically discrete units suitable as unitary dosages for animal and human subjects, each unit containing a predetermined quantity of active

material calculated to produce the desired biological effect in association with required pharmaceutical means.

The low cost and ready accessibility of the compositions of this invention make them particularly promising for applications in veterinary medicine in which field their utilities are comparable to those in human medicine.

The novel compounds of this invention are readily prepared by reaction of an amine of the formula:

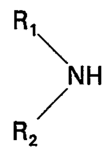

with an anhydride or an ester of a fluorinated lower aliphatic carboxylic acid to produce an amide compound of the formula:

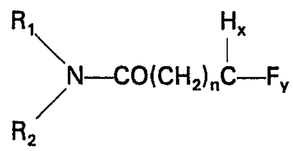

wherein n is 0 or an integer of from 1—8 inclusive, x is 0 or 1, and y is 2 or 3 provided that the sum of x and y is 3.

The resulting amide compound is then reduced to the desired amine product by treatment with a reducing agent such as borane in a suitable aprotic solvent such as tetrahydrofuran at a temperature of from 0°C to the reflux temperature of the reaction mixture of a period of from 1—24 hours.

Examples of such amines are

N-dodecyl-2,2,2-trifluoroethylamine;
N-octyl-2,2,2-trifluoroethylamine;
N-decyl-2,2,2-trifluoroethylamine;
N-hexadecyl-2,2,2-trifluoroethylamine;
N-octadecyl-2,2,2-trifluoroethylamine;
N-dodecyl-2,2-difluoroethylamine.

The following Examples illustrate the preparations of novel compounds and compositions in accordance with the present invention and of certain intermediate products useful in such preparations.

Example 1
N-Dodecyl difluoroacetamide

One equivalent difluoroethylacetate and one equivalent dodecylamine are stirred at room temperature in acetonitrile 16 hours. The solvent is removed to give a white wax, which is chromatographed on silica gel in $CHCl_3$/EtOAc (15:1), $R_f$=0.7. NMR ($CDCl_3$, $\delta$) 7.2 b-s (1H); 5.9 t (2H) J=26; 3.35 m (2H); 1.3 s (20H); 0.9 m (3H). Other compounds prepared this way, with similar NMR, acceptable Mass Spectra and TLC: N-propyl-difluoroacetamide.

Example 2
N-Dodecyl-trifluoroacetamide

Trifluoroacetic anhydride (21 ml; 1.5 equivalents) is added dropwise to a stirred solution of 17.9 g dodecylamine (1 equivalent) and 13.5 ml triethylamine (1 equivalent) in 100 ml $CH_2Cl_2$ at 0°C over 30 minutes, and the mixture stirred at room temperature 30 minutes. It is washed with water, 1M $H_3PO_4$, brine, dried over $MgSO_4$, filtered, stripped to yield 26.01 g product (96%). TLC in cyclohexane EtOAc (10:1) gives one spot at $R_f$—0.5 and no starting material ($R_f$—0.05). NMR ($\delta$, $CDCl_3$): 6.19 bs (1H); 3.30 q, J=4 (2H); 1.20 s (20H); 0.92 m (3H).

The following similar compounds were all prepared in the same manner, all with similar NMR, acceptable microanalyses, mass spectra, and single spot on TLC: N-propyl-trifluoroacetamide, N-pentyl-trifluoroacetamide, N-octyl-trifluoroacetamide, N-decyl-trifluoroacetamide, N-hexadecyl-trifluoroacetamide, N-octadecyl-trifluoroacetamide.

Example 3
N-Dodecyl-2,2,2-trifluoroethylamine

A solution of 8 mM $BH_3$ in THF (8 ml) is added dropwise to a solution of 1.12 g (4 mM) dodecyl-trifluoroacetamide in 6 ml THF at 0°C under $N_2$. The mixture is refluxed 16 hours, cooled in ice, and 6 ml concentrated HCl added slowly. The THF is distilled off at atmosphere pressure, the mixture cooled in ice, and solid NaOH added slowly with cooling until alkaline to pH paper. The aqueous solution is

then extracted three times with hexane. The combined organic layers are washed with water, dried over $K_2CO_3$, filtered, stripped to give 2.0 g crude product. It is then chromatographed in cyclohexane/EtOAc (10:1) on silica gel ($R_f$=0.4) to give 870 mg oil which is distilled at 0.3 mm, b.p. 135°C, to give 405 mg pure product, 38% yield. NMR ($CDCl_3$, $\delta$): 3.22 q, J=10 (2H); 2.79 m (2H); 1.40 s (20 H); 0.93 m (3H). IR shows no carbonyl bands.

Other compounds made this way, all with similar NMR and other spectral data:

N-octyl-2,2,2-trifluoroethylamine;
N-decyl-2,2,2-trifluoroethylamine;
N-hexadecyl-2,2,2-trifluoroethylamine;
N-octadecyl-2,2,2-trifluoroethylamine;
N-dodecyl-2,2-difluoroethylamine.

Example 4
Contraceptive formulations

A suitable formulation is prepared by mixing the lysosomotropic amine and other ingredients employed in the following proportions (in percentages):

| | |
|---|---|
| Benzyldimethyl [2-[2-(p-1,1,3,3-tetramethylbutylphenoxy)ethoxyl]ethyl]-ammonium chloride (benzethonium chloride, U.S.P.) | 0.2 |
| N-dodecyl-2,2,2-trifluoroethylamine | 8.0 |
| Myristic acid | 2.0 |
| Stearic acid | 4.0 |
| Triethanolamine | 2.0 |
| Glyceryl monosterate | 3.0 |
| Polyoxyethylene (20) sorbitan mono-oleate | 3.0 |
| Polyoxyethylene (20) sorbitan monolaurate | 3.0 |
| Polyvinylpyrrolidone | 1.0 |
| Polyethylene glycol (average molecular weight, 600) | 1.4 |
| Deionized water | 72.4 |
| | 100.0 |

The procedure is repeated using as the active ingredient, in place of N-dodecyl-2,2,2-trifluoroethylamine, a similar percentage of any of the compounds prepared in accordance with Example 3.

Example 5
Capsule formulation

| | |
|---|---|
| N-dodecyl-2,2,2-trifluoroethylamine | 25 g |
| Stearic acid (U.S.P. triple pressure) | 125 g |
| Pluronic F-68 | 7.5 g |
| Corn starch | 125 g |

('Pluronic' is a registered trade mark)

The stearic acid and Pluronic are united in a vessel and melted using a water bath at 60—65°C. The heating is discontinued and the N-dodecyl-2,2,2-trifluoroethylamine is dispersed into the mixture and the corn starch is added with stirring which is continued until the mixture cools to ambient temperature. The mixture is reduced to granules by screening and placed in a number 0 hard gelatin containing 282.5 mg of total solids and 25 mg of N-dodecyl-2,2,2-trifluoroethylamine per capsule.

This procedure is repeated using as the active ingredient any of the compounds prepared in accordance with Example 3 with resultant production of pharmaceutical formulations of the present invention.

## Claims

1. A pharmaceutical composition comprising a pharmaceutical carrier and a lysosomotropic amine compound having a pK of from 4.5—9 and of the formula:

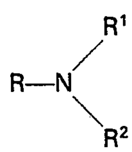

in which R is a $C_{8-30}$ alkyl group, $R^1$ a hydrogen atom or an alkyl group and $R^2$ is a $C_{2-3}$ fluorinated alkyl group in which the fluorine atom(s) is/are attached to its terminal carbon atom.

2. A novel lysosomotropic amine detergent having a pK of 4.5 to 9 and having the formula set forth in Claim 1.

3. A method of preparing a compound as claimed in Claim 2, comprising reacting an amine of formula

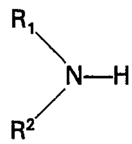

with an anhydride or ester of a suitable fluorinated alkanoic acid to produce an amide of formula

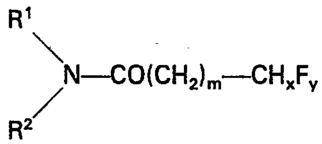

in which *m* is 0 or 1, *x* is 0 or 1 and y is 3—*x*, and reducing the latter with a reducing agent in an aprotic solvent at a temperature of from 0°C to reflux for from 1 to 24 hours.

4. A composition as claimed in Claim 1 or a compound as claimed in Claim 2 for use in the inhibition or alteration of the function of lysosome-bearing cells.

**Patentansprüche**

1. Eine pharmazeutische Zusammensetzung, enthaltend einen pharmazeutischen Träger und eine lysosomotropische Aminverbindung mit einem pK-Wert von 4,5 bis 9 entsprechend der Formel

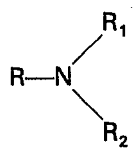

worin R eine $C_{8-30}$-Alkylgruppe bedeutet, $R_1$ ein Wasserstoffatom oder eine Alkylgruppe darstellt und $R_2$ eine $C_{2-3}$-fluorierte Alkylgruppe darstellt, worin das Fluoratom bzw. die Fluoratome an deren endständiges Kohlenstoffatom gebunden ist bzw. sind.

2. Ein neues lysosomotropisches Amin-Detergens mit einem pK-Wert von 4,5 bis 9 und mit der in Anspruch 1 angegebenen Formel.

3. Ein Verfahren zur Herstellung einer Verbindung nach Anspruch 2, umfassend die Umsetzung eines Amins der Formel

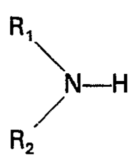

mit einem Anhydrid oder Ester einer geeigneten fluorierten Alkansäure unter Ausbildung eines Amids der Formel

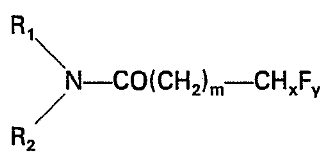

worin m den Wert Null oder 1 aufweist, x den Wert Null oder 1 hat und y den Wert 3—x aufweist, und die Reduktion des letztgenannten mit einem Reduktionsmittel in einem aprotischen Lösungsmittel bei einer Temperatur von 0°C bis zur Rückflußtemperatur während 1 bis 24 h.

4. Eine Zusammensetzung nach Anspruch 1 oder eine Verbindung nach Anspruch 2 zur Anwendung in der Inhibierung oder Änderung der Funktion von Lysosomen tragenden Zellen.

**Revendications**

1. Composition pharmaceutique comprenant un support pharmaceutique et un composé d'amine lysosomotrope ayant un pK allant de 4,5 à 9 et de formule:

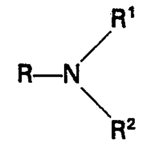

où R est un groupe alcoyle en $C_8$ à $C_{30}$, $R^1$ est un atome d'hydrogène ou un groupe alcoyle et $R^2$ est un groupe alcoyle fluoré en $C_2$ à $C_3$ où le(s) atome(s) de fluor est (sont) attaché(s) à l'atome de carbone terminal.

2. Nouveaux détergents à l'amine lysosomotrope ayant un pK de 4,5 à 9 et ayant la formule exposée dans la revendication 1.

3. Procédé de préparation d'un composé selon la revendication 2, dans lequel on fait réagir une amine de formule

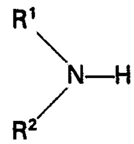

avec un anhydride ou ester d'un ester alcanoïque fluoré approprié pour produire un amide de formule

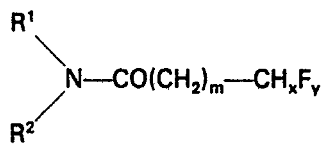

où *m* vaut 0 ou 1, *x* vaut 0 ou 1 et *y* vaut 3—*x*, et on réduit ce dernier avec un agent réducteur dans un solvant aprotique à une température allant de 0°C au reflux pendant 1 à 24 h.

4. Composition selon la revendication 1 ou composé selon la revendication 2 aux fins d'application à l'inhibition ou à l'altération de la fonction des cellules portant des lysosomes.